# EUROPÄISCHE PATENTSCHRIFT

(11) **EP 1 598 094 B1**
(45) Veröffentlichungstag und Bekanntmachung des Hinweises auf die Patenterteilung: **07.03.2007**
(21) Anmeldenummer: 05010450.4
(22) Anmeldetag: 13.05.2005
(51) Int. Cl.: A61N 5/10

(54) **Strahlzuteilungsverfahren für medizinische Teilchenbeschleuniger**
Beam allocation method for medical particle accelerators
Méthode d'allocation de faisceau pour accélérateurs médicaux de particules

(30) Priorität: 19.05.2004 DE 102004025263; 02.06.2004 DE 102004027071
(43) Veröffentlichungstag der Anmeldung: 23.11.2005
(73) Patentinhaber: Gesellschaft für Schwerionenforschung mbH, 64291 Darmstadt (DE)
(72) Erfinder: Naumann, Jakob, Dr., 64285 Darmstadt (DE); Poppensieker, Klaus, Dr., 64287 Darmstadt (DE)
(74) Vertreter: Boeters, Hans Dietrich

(56) Entgegenhaltungen:
- EP-A- 0 986 070
- EP-A- 1 454 654
- WO-A-00/48673
- US-A- 5 260 581
- PEDRONI E ET AL: "THE 200-MEV PROTON THERAPY PROJECT AT THE PAUL SCHERRER INSTITUTE: CONCEPTUAL DESIGN AND PRACTICAL REALIZATION" MEDICAL PHYSICS, AMERICAN INSTITUTE OF PHYSICS. NEW YORK, US, Bd. 22, Nr. 1, Januar 1995 (1995-01), Seiten 37-53, XP000505145 ISSN: 0094-2405

## Beschreibung

Die Erfindung betrifft ein Strahlzuteilungsverfahren.

Eine Strahlzuteilungsvorrichtung und ein Strahlzuteilungsverfahren für medizinische Teilchenbeschleuniger sind aus den Druckschriften US 5,260,581 und US 5,895,926 bekannt.

Aus der Druckschrift US 5,260,581 ist ein Verfahren zur Bestätigung einer Behandlungsraumauswahl in einem Strahlentherapiesystem bekannt. Das Verfahren vergleicht Anfragesignale der Behandlungsräume mit einem Signal zu einer Bestrahlungspfadgestaltung von einem Kontrollraum aus, der den Pfad des Strahls, der von einem Beschleuniger zu einem der Behandlungsräume transportiert wird, steuert. Bei Übereinstimmung von Anfrage- und Strahlpfadsignalen wird der Transport zu einem ausgesuchten Bearbeitungsraum autorisiert. Dazu weist der Strahlpfad für jeden der Behandlungsräume Ablenkmagnete auf, die den beschleunigten Strahl von einer Transportstrecke in einem ersten Zustand des Ablenkmagneten zu der Bearbeitungsstation ablenken und in einem zweiten Zustand der Ablenkmagneten den Strahl in einen behandlungsfreien Auffangraum ablenken.

Aus der Druckschrift US 5,895,926 ist ein Verfahren und eine Vorrichtung für die Strahlsicherheit in einer Strahlbehandlungsanlage bekannt. Das System überwacht und steuert die Strahlungssteuerungssysteme, um sie vor Fehlpfaden und/oder mehrfach Pfadbedingungen, was zu einem unbeabsichtigten Strahlungsvorgang führen kann, zu schützen. Dazu wird bei dem Verfahren die Konfiguration der Strahlpfadsignale mit Signalen einer angeforderten Strahlkonfiguration verglichen, um die Übereinstimmung zu prüfen. Dabei wird besonders darauf geachtet, dass keine Überhitzungen auftreten und nicht autorisiertes Personal in die Pfadbedingungen eingreifen kann.

Bei derartigen Verfahren wird eine Übertragung der Strahlung auf den Behandlungsraum nur in autorisierten Fällen ermöglicht. Für den Abbruch einer Behandlung hat jeder der Behandlungsräume mit seinem Kontrollraum einen direkten, ihm zugeordneten Zugriff zu einzelnen Ablenkmagneten in der Transportstrecke des Ionenstrahls zu den Behandlungsräumen. Die Trägheit der Ablenkungsmagnete, insbesondere ihr Selbstinduktionsverhalten, ermöglicht einen Abbruch der Strahlversorgung innerhalb von etwa 500 ms. Für eine Ionenstrahlbehandlung jedoch, die nicht, wie im genannten Stand der Technik, auf flächiger Ausleuchtung des Tumorgewebes basiert, sondern auf einem punktförmigen Abtasten des Tumorgewebes mittels eines scannenden Verfahrens (z.B. Rasterscan-Verfahren), sind derartige Abschaltzeiten durch Ablenkmagnete in Transportstrekken des hochbeschleunigten Ionenstrahls nicht akzeptabel.

Ein für die Zwecke der Strahlentherapie tauglicher Ionen- oder Protonenstrahl beinhaltet zwangsläufig eine ionisierende Strahlung im Sinne der Strahlenschutzverordnung und ist damit auch potenziell für den Menschen gefährlich. Daher muss bei diesen Anlagen vollständig gewährleistet sein, dass der Strahl des Beschleunigers nur zu einem definierten Zeitpunkt und auf definierte Weise einen Bestrahlungsraum erreichen kann. Für eine punktförmige scannende Bestrahlung muss deshalb die Möglichkeit bestehen, innerhalb kürzester Zeit von wenigen hundert Mikrosekunden den Strahlvorgang abzubrechen und einen "Spillabbruch" auszulösen, wenn sich bspw. die vom Beschleuniger gelieferte Strahlqualität verschlechtert.

Andererseits ergibt sich aus beschleunigungstechnischer Sicht die Anforderung, dass Elemente der Strahlführung, die einen derart schnellen Strahlabbruch ausführen könnten und somit ein verbessertes "Spillabbruchsystem" bilden könnten, nicht für jeden Bestrahlungs- bzw. Kontrollraum separat vorhanden sind oder sein können. In einer Strahlentherapieanlage ist nämlich der eigentliche Beschleunigungsbereich (z.B. in Form eines Synchrotrons, Cyklotrons oder Linearbeschleunigers; im Folgenden wird der Begriff "Beschleunigungsbereich" auch für die unmittelbar auf den Beschleunigungsbereich folgenden, für mehrere Bestrahlungsräume gemeinsamen, Komponenten verwendet), der über ein derartiges ausreichend schnelles "Spillabbruchsystem" verfügt, nicht mehrfach vorhanden, sondern versorgt mehrere Behandlungsräume, sodass nicht jedem Behandlungsraum ein eigener Beschleunigungsbereich (z.B. ein eigenes Synchrotron) für einen schnellen "Spillabbruch" zur Verfügung steht.

Weltweit existieren einige wenige medizinische Strahlungseinrichtungen mit einem Teilchenstrahl für mehrere Bestrahlungsräume, jedoch verwendet keine dieser Anlagen ein scannendes Verfahren, bei dem ein punktförmiger Ionenstrahl ein Tumorgewebe abtastet mit den entsprechend hohen Anforderungen an das Zeitverhalten eines "Spillabbruchsystems". Typischerweise sind die für Sicherheitsfunktionen verwendeten Elemente im Stand der Technik, nämlich die Ablenkmagnete der einzelnen Bestrahlungsplätze für scannende Verfahren zu langsam. Eine Nutzung von zwangsläufig gemeinsamen Elementen, wie dem oben erwähnten Beschleunigungsbereich, ist mit den bisher bekannten Methoden und den bisher bekannten Strahlzuteilungsvorrichtungen nicht möglich. Eine unkontrollierte gleichzeitige Einflussnahme mehrerer Behandlungsräume erlaubt keinen geregelten Betrieb und ist potentiell für den Patienten gefährlich.

Aufgabe ist es, eine Strahlzuteilungsvorrichtung zu schaffen, die sicherheitsrelevant im Sinne des Medizinproduktgesetzes einen gleichzeitigen zugriff mehrerer Kontrollräume entsprechender Bestrahlungsräume oder Qualitätssicherungsräume auf gemeinsam genutzte Komponenten eines Teilchenbeschleunigers verhindert und insbesondere eine gleichzeitige Einflussnahme mehrerer Kontrollräume auf das "Spillabbruchsystem" eines Beschleunigungsbereichs ausschließt. Ferner ist es die Aufgabe, eine Schaltungseinheit zu schaffen, welche die Strahlhoheit für mehrere gleichberechtigte Kontrollräume sicherheitsrelevant regelt.

Aufgabe der Erfindung ist es, die Strahlhoheit im klinischen Betrieb einer vorbestimmten Reihenfolge für eine optimale Auslastung des Beschleunigers, sowie auch eine spontane Reaktion auf einen Notfall zu ermöglichen. Schließlich ist es Aufgabe, eine Schaltungseinheit zu schaffen, die mit wenigen sicherheitsrelevanten Komponenten die Strahlhoheit vergibt, um den Aufwand für eine Zertifizierung im Rahmen eines EU-Konformitätsbewertungsverfahrens zum Zwecke der Zulassung als Medizinprodukt gemäß Medizinproduktgesetz gering zu halten.

Diese Aufgabe wird mit dem Gegenstand des unabhängigen Anspruchs gelöst. Vorteilhafte Weiterbildungen der Erfindung ergeben sich aus den abhängigen Ansprüchen.

Eine Strahlzuteilungsvorrichtung für medizinische Teilchenbeschleuniger wird offenbart. Der Teilchenbeschleuniger weist mindestens einen Beschleunigungsbereich, eine Transportstrecke und Ablenkkomponenten, über welche mehrere jeweils einen Kontrollraum aufweisende Behandlungsräume mit einem Teilchenstrahl aus Ionenpaketen versorgt werden.

Die offenbarte Strahlzuteilungsvorrichtung weist eine Arbitrierungseinheit mit Schaltlogik, Überwachungseinheit und Ablaufsteuerung auf, die über Signalleitungen mit einem Spillabbruchsystem, das mindestens zwei schnelle Spillabbruchelemente im Beschleunigungsbereich oder der allen Bestrahlungsplätzen gemeinsamen Strahlführung aufweist, elektrisch in Verbindung steht. Dazu stellt die Strahlzuteilungsvorrichtung einen direkten Zugriff des Kontrollraums des bestrahlungsaktiven Bestrahlungsraumes auf das Spillabbruchsystem des Beschleunigungsbereichs bereit, um einen Teilchenstrahlabbruch bei Gefahr innerhalb von Mikrosekunden zu realisieren.

Die offenbarte Strahlungszuteilungsvorrichtung hat den Vorteil, dass sie nicht nur die herkömmlichen Zugriffsmöglichkeiten und Eingriffsmöglichkeiten auf dem jeden Bestrahlungsraum zugeordneten Ablenkmagneten ermöglicht, sondern zusätzlich einen um mehrere Größenordnungen schnelleren Abbruch der Strahlbehandlung bei Gefahr ermöglicht, indem es die Strahlhoheit und den Zugriff auf das Spillabbruchsystem des Beschleunigungsbereichs dem Kontrollraum des jeweils bestrahlungsaktiven Behandlungsraumes zuteilt. Somit wird der Zugriff auf das Spillabbruchsystem sicherheitsrelevant durch eine Instanz, nämlich der Arbitrierungseinheit, geregelt, ein schnellerer Strahlabbruch ermöglicht und die gleichzeitige Einflussnahme mehrerer Kontrollräume auf das Spillabbruchsystem verhindert. Gleichzeitig ermöglicht die offenbarte Strahlenzuteilungsvorrichtung, dass die Strahlhoheit in der Reihenfolge der bestrahlungsaktiven Kontrollräume vergeben wird und innerhalb dieser Zugriffszeit ermöglicht wird, dass eine spontane Reaktion auf einen Notfall erfolgen kann. Ferner ist durch die Konzentration der Vergabe der Zugriffsberechtigung auf eine Arbitrierungseinheit mit Schaltlogik, Überwachungseinheit und Ablaufsteuerung die Anzahl der sicherheitsrelevanten Komponenten äußerst gering, sodass der Aufwand für eine Zertifizierung im Rahmen eines EU-Konformitätsbewertungsverfahrens zum Zwecke der Zulassung als Medizinprodukt gemäß Medizinproduktgesetz gering bleibt.

Das Spillabbruchsystem weist als Spillabbruchelement mindestens einen Spillabbruchmagneten auf. Darüber hinaus hat die Strahlzuteilungsvorrichtung Zugriff auf einen Exiter des Beschleunigungsbereichs.

In einer bevorzugten offenbarten Variante weist jeder Kontrollraum eines Behandlungsraumes und/oder eines Qualitätssicherungsraumes und/oder eines Teilchenbeschleunigers, eine Requestsignalleitung zu der Schaltlogik der Arbitrierungseinheit zur Anfrage einer Übertragung einer Strahlhoheit auf den Kontrollraum mit Zugriffsberechtigung auf den Exciter und/oder den Spillabbruchmagneten des Beschleunigungsbereichs auf. Diese offenbarte Variante hat den Vorteil, dass vorbereitend die Strahlzuteilungsvorrichtung aufgrund der Requestsignalleitung zu der Schaltlogik der Arbitrierungseinheit eine Reihenfolge unter gleichberechtigten. Kontrollräumen und einem zeitlichen Ablauf der Zugriffsberechtigungen vorbereiten kann.

Über eine weitere Signalleitung, der so genannten Grantsignalleitung zu dem Kontrollraum wird nach Rückkopplung mit dem Spillabbruchsystem des Beschleunigungsbereichs die Anfrageabgabe und die Einreihung in eine Warteschlange dem Kontrollraum bestätigt. Eine dritte Signalleitung zwischen Kontrollraum und Schaltlogik der Arbitrierungseinheit, eine so genannte Clearsignalleitung dient dazu, aktiv vom Kontrollraum aus das Ende oder den zeitweisen Verzicht auf eine Strahlhoheit zu signalisieren und damit aktiv die Verfügung der Strahlhoheit an die Arbitrierungseinheit zurückzugeben

Über eine vierte Leitung ist der Kontrollraum mit der Schaltlogik der Arbitrierungseinheit verbunden, um über diese so genannte "Spillpauseleitung" eine Unterbrechung der Strahlzufuhr auszulösen. Dabei wird der Abbruch der Bestrahlung durch unmittelbaren zugriff des Kontrollraumes auf den Exciter und/oder den Spillabbruchmagneten des Beschleunigungsbereichs bewirkt. Diese Spillpauseleitung hat somit den Vorteil für den Kontrollraum des bestrahlungsaktiven Behandlungsraumes, dass innerhalb von wenigen Mikrosekunden ein Strahlabbruch erfolgt. Spillpausen werden während einer Bestrahlung nach scannenden Verfahren regulär erzeugt, stellen also keinen Fehlerfall dar.

Eine fünfte Verbindung zwischen Kontrollraum und Schaltlogik der Arbitrierungseinheit besteht in einer Interlockleitung, über die ein Abbruch der Bestrahlung durch unmittelbaren zugriff des Kontrollraumes auf den Exciter und/oder den Spillabbruchmagneten des Beschleunigungsbereichs unter Beibehaltung einer Zugriffsreservierung erfolgt. Diese Interlockleitung wird immer dann aktiviert, wenn ein akuter, jedoch relativ kurzfristiger Notfall eingetreten ist, wie bspw. eine unerwartete Verschiebung der Position der gemessenen Strahlposition im Bestrahlungsraum.

Neben diesen fünf Verbindungen zwischen Kontrollraum und Schaltlogik der Arbitrierungseinheit ist es in einer weiteren offenbarten Variante vorgesehen, dass die Kontrollräume unabhängig von der Arbitrierungseinheit seriell mit einer potenzialfreien Interlockleitung zu dem Spillabbruchsystem des Beschleunigungsbereichs Zugriff haben. Dazu sind die potenzialfreien Schalter aller Kontrollräume seriell geschaltet und geschlossen, sodass bei Betätigung des Schalters durch einen der Kontrollräume ein unmittelbarer Spillabbruch über diesen redundanten Signalpfad erfolgen kann. Durch die serielle Schaltung der potenzialfreien Interlockleitung wird erreicht, dass unabhängig von der Strahlzuteilung jeder Bestrahlungsplatz einen Abbruch der Bestrahlung erzwingen kann, wenn der primäre Signalpfad des Spillabbruchsystems oder die Arbitrierungseinheit selbst versagt.
Entsprechend den Verbindungen zwischen Schaltlogik und Kontrollräumen bestehen entsprechende elektrische Verbindungen zwischen der Schaltlogik der Arbitrierungseinheit und dem Spillabbruchsystem des Beschleunigungsbereichs. Da jeweils eine Requestsignalleitung, eine Grantsignalleitung, eine Spillpauseleitung und eine Interlockleitung, sowohl mit dem Exciter als auch mit dem Spillabbruchmagneten vorhanden sind, ist die Anzahl der Signalleitungen zwischen Schaltlogik der Arbitrierungseinheit und dem Spillabbruchsystem doppelt ausgelegt, womit die Systemsicherheit weiter erhöht wird.

Die Überwachungseinheit der Arbitrierungseinheit empfängt vorzugsweise über Eingangssignalverbindungen von der Schaltlogik und der Ablaufsteuerung Signale und ist über Ausgangsverbindungen mit dem Spillabbruchsystem des Beschleunigungsbereichs verbunden. Mit diesen Leitungen überprüft die Überwachung der Arbitrierungseinheit, ob mehrere Anforderungsinstanzen ein Grant-Signal erhalten haben oder für ein Strahlziel ein Grant-Signal ohne zugehöriges Request-Signal vorliegt, oder ob für ein Strahlziel ein Grant-Signal vorliegt, obwohl dieses Strahlziel nicht der Vorgabe der Ablaufsteuerung entspricht. In diesen genannten Fällen wird unmittelbar ein Spillabbruch normalerweise über einen redundanten Signalpfad ausgelöst.

Bei einer weiteren offenbarten Variante ist ein Scheduler mit der Schaltlogik der Arbitrierungseinheit verbunden und über die Ablaufsteuerung steuert die Arbitrierungseinheit das Abarbeiten einer im Scheduler erstellten Warteschlange von Bestrahlungsanforderungen. Ein derartiger Scheduler kann einen Computer oder eine Anzahl von Mikroprozessoren aufweisen. Er dient der Optimierung der Eingangsdaten, prüft das Vorhandensein von Bestrahlungsplänen und die Korrektheit der Strahlvergabe. Darüber hinaus unterstützt der Scheduler die Darstellung der Warteschlange in allen Kontrollräumen, liefert eine Fortschrittsanzeige und sorgt für Warnmeldungen und die Aktualisierung der Warteschlange. Außerdem prüft er die Verfügbarkeit von Anforderungsinstanzen bzw. die Verfügbarkeit der Kontrollräume, liefert eine Rückmeldung für die Terminierung und regelt Zugriffsrechte der unterschiedlichen Kontrollräume. Trotz dieser vielfältigen Aufgaben des Schedulers gehört er mit seinen Verbindungen nicht zum sicherheitsrelevanten Bestandteil der erfindungsgemäßen Strahlzuteilungsvorrichtung, sondern ist vielmehr eine Ergänzung, sodass bei Ausfall des Schedulers oder ein nicht Vorhandensein eines Schedulders in einer Strahlentherapieanlage die Arbitrierungseinheit ein Verfahren anwendet, bei dem die Kontrollräume und damit die Anforderungseinheiten reihum die Gelegenheit einer Reservierung eines Ionenstrahls vorzunehmen, erhalten.

Vorzugsweise ist der Exciter eine Hochfrequenzresonanzkomponente für Ionenpakete im Einkopplungsbereich eines als Beschleunigungsbereich dienenden Synchrotrons, sodass durch Verstellen bzw. Fehlanpassen der Resonanzabstimmung ein schneller Strahlabbruch in Mikrosekunden am Exciter ausgelöst werden kann. Dem gegenüber ist der Spillabbruchmagnet ein Strahlführungsmagnet im Auskopplungsbereich eines als Beschleunigungsbereich dienenden Synchrotrons, mit dem ein Auskoppeln des Strahls aus dem Beschleunigungsbereich in wenigen Mikrosekunden verhindert werden kann. Beide Komponenten, die praktisch gemeinsam für alle Kontrollräume zur Verfügung stehen, haben den Vorteil, dass sie einen Strahlabbruch um mehrere Zehnerpotenzen gegenüber herkömmlichen Strahlzuteilungsvorrichtungen verkürzen können und somit eine Therapieanlage, die auf scannenden Verfahren (z.B. Rasterscan-Verfahren) basiert, mit einer erhöhten Strahlenschutzsicherheit ausstatten können.

In einer weiteren offenbarten Variante weist die Strahlzuteilungsvorrichtung eine Steuerung und Verwaltung der Zuteilung eines Teilchenstrahls eines Bestrahlungssystems für ein Rasterscan-Verfahren mit einem Ionenschreibstrahl einer Ionenstrahlabtastvorrichtung für ein Zielvolumen auf. Eine derartige Anlage wird gegenwärtig in Heidelberg errichtet und steht als Entwicklungs- und Forschungsanlage bisher nur in Darmstadt bei der Gesellschaft für Schwerionen zur Verfügung.

Das erfindungsgemäße Strahlzuteilungsverfahren für medizinische Teilchenbeschleuniger weist die nachfolgenden verfahrensschritte auf:

Zunächst wird eine Reservierung der Teilchenstrahlhoheit, insbesondere eine Reservierung eines direkten Zugriffs auf ein Spillabbruchsystem des von allen Behandlungsräumen benötigten Beschleunigungsbereichs für eine geplante Dauer einer Bestrahlung durch einen der Kontrollräume beantragt. Anschließend wird eine sicherheitsrelevante Reservierung des Spillabbruchsystems durch eine elektronische Schaltungslogik einer Arbitrierungseinheit durchgeführt. Schließlich erfolgt ein Mitteilen der Reservierung an jeden Kontrollraum unter Zulassen nur eines Kontrollraums für einen Strahlabbruch über das Spillabbruchsystem.

Ferner wird ein von der Arbitrierungseinheit unabhängiger unmittelbarer und redundanter Signalpfad zum Spillabbruchsystem des Beschleunigungsbereichs für die Kontrollräume bereitgehalten, wobei die Schaltelemente der Kontrollräume in Reihe geschaltet sind. Dabei entscheidet die Arbitrierungseinheit bei gleichzeitiger Strahlreservierungsanforderung mehrerer Kontrollräume, welcher Kontrollraum eines Bestrahlungsplatzes die Reservierung vornehmen kann. Trotz Verlust einer Reservierung kann von jedem Kontrollraum über den redundanten Signalpfad ein Spillabbruch ausgelöst werden.

Dieses Verfahren hat den Vorteil der doppelten Sicherheit, dadurch, dass einerseits über übergeordnete Instanzen der Arbitrierungseinheit ein Spillabbruch nur dann durchgeführt werden kann, wenn die Kontrolleinheit bzw. der Kontrollraum eine Anfrage bzw. eine Request über ihre Requestleitung aktiv durchgeführt hat und in Form einer Rückmeldung über die Grantleitung ein Grantsignal empfangen hat. Damit ist der Vorteil verbunden, dass nicht unvorbereitet und unkontrolliert einem Kontrollraum eine Spillabbruchmöglichkeit eingeräumt wird. Somit wird auch vermieden, dass gleichzeitig mehrere Kontrollräume einen Spillabbruch auslösen können, zumal über die Arbitrierungseinheit sichergestellt ist, dass nur der Kontrollraum eines bestrahlungsaktiven Bestrahlungsraumes Zugriff auf das schnelle Strahlabbruchsystem des Beschleunigungsbereichs erhält. Der redundante Signalpfad, über den unabhängig von der Arbitrierungseinheit ein Kontrollraum einen Spillabbruch auslösen kann, ist durch serielle Anordnung der Schaltelemente für die Kontrollräume vor einem gleichzeitigen gegenläufigen Zugriff mehrerer Kontrollräume auf das Spillabbruchsystem geschützt - jeder Bestrahlungsplatz ist also auf diesem wege jederzeit in der Lage, den Strahl abzuschalten, das Wiedereinschalten erfordert hingegen das Zusammenwirken aller Bestrahlungsplätze, die einen Abbruch verursacht haben.

Bei einem weiteren Durchführungsbeispiel des Verfahrens wird die Strahlhoheit erst dann von einem aktiven Kontrollraum zu einem anderen Kontrollraum übergeben, wenn der aktuelle Kontrollraum die Kontrolle über den Teilchenstrahl aktiv abgibt. Das hat den Vorteil, dass jeglicher unvorbereiteter automatischer Übergang der Strahlhoheit auf einen anderen Kontrollraum nicht möglich ist. Der Beschleunigungsbereich ist somit vor einem gleichzeitigen Zugriff mehrerer Kontrollräume auf das Spillabbruchsystem geschützt. Darüber hinaus besteht die Möglichkeit, dass das Spillabbruchsystem des Beschleunigungsbereichs für eine kurzzeitige Strahlabschaltung genutzt wird. Dieses geschieht über die Interlockleitung und hat zur Folge, dass, sobald der Fehler behoben ist, die Bestrahlung fortgesetzt werden kann, ohne dass der Kontrollraum die Strahlhoheit übergibt, oder zwischenzeitlich die Strahlhoheit an einen anderen Kontrollraum übergeht. Auch ist es vorgesehen, dass die Arbitrierungseinheit ermöglicht, dass ein Kontrollraum für den Teilchenbeschleuniger eine Reservierung für das Spillabbruchsystem vornimmt. Dem Kontrollraum des Beschleunigers sind alle Bereiche der Strahlführung zugänglich, für die gewährleistet ist, dass sich dort kein Patient aufhält.

Da der Kontrollraum für den Teilchenbeschleuniger grundsätzlich mit sämtlichen Komponenten des Beschleunigungsbereichs und in Verbindung steht bzw. auf diesen einen Zugriff hat, ist das Vorsehen einer Reservierung eines Zugriffs auf das Spillabbruchsystem für den Kontrollraum des Teilchenbeschleunigers dennoch sinnvoll, um die Anforderung des Kontrollraums des Teilchenbeschleunigers in die warteschlange, die durch die Arbitrierungseinheit abgearbeitet wird, aufnehmen zu können und einen optimalen Einsatz der Bestrahlungsanlage zu gewährleisten. Ferner ist es vorgesehen, dass der Status aller Reservierungen, Zugriffsberechtigungen und Quittungen durch eine Überwachungseinheit der Arbitrierungseinheit überprüft wird und bei Inkonsistenz eine Abschaltung über den redundanten Signalpfad durchgeführt wird. Das hat den Vorteil, dass keine unvorhersehbaren Zugriffskollisionen auftreten können, da die Überwachungseinheit den Status aller Reservierungen, Zugriffsberechtigungen und Quittungen überprüft und sicherheitshalber einen Strahlabbruch verursacht, bevor ein größerer Schaden entsteht.

Ferner ist es vorgesehen, durch ein nicht sicherheitsrelevantes Mikroprozessorsystem, das als Scheduler fungiert, eine Abfolge der Vergabe von Reservierung des Spillabbruchsystems der Arbitrierungseinheit mitzuteilen. Bei einem Fehlen des Mikroprozessorsystems oder bei Fehlen von Vergaben ist es vorgesehen, dass die Arbitrierungseinheit den Kontrollräumen reihum die Gelegenheit gibt, eine Reservierung vorzunehmen.

Mit diesem Verfahrensschritt wird sichergestellt, dass in jedem Fall die Arbitrierungseinheit eine ausreichende sicherheitsrelevante Versorgung der Kontrollräume mit einem Zugriff auf das schnelle Spillabbruchsystem ausstattet.

Die Erfindung wird nun anhand der beigefügten Figuren näher erläutert.
- Figur 1: zeigt ein Schema eines Blockschaltbildes einer Strahlzuteilungsvorrichtung;
- Figur 2: zeigt eine Prinzipskizze eines Teilchenbeschleunigers mit Komponenten, auf die von der Strahlzuteilungsvorrichtung zugegriffen wird;
- Figur 3: zeigt eine Prinzipskizze einer Requestschaltung zum Verarbeiten von Requestsignalen über Requestleitungen;
- Figur 4: zeigt eine Prinzipskizze einer Grantschaltung zur Verarbeitung von Grantsignalen über Grantleitungen;
- Figur 5: zeigt eine Prinzipskizze einer Spillpause-Schaltung zur Verarbeitung von Spillpausesignalen über Spillpauseleitungen;
- Figur 6: zeigt eine Prinzipskizze einer Interlockschaltung zur Verarbeitung von Interlocksignalen über Interlockleitungen:
- Figur 7: zeigt ein Schema eines Flussdiagramms einer Ablaufsteuerung einer Arbitrierungseinheit.
- Figur 8: zeigt eine Prinzipskizze der Serienschaltung von potenzialfreien Kontakten der Kontrollräume für einen Spillabbruch über einen Exciter des Beschleunigungsbereichs.
- Figur 9: zeigt eine Prinzipskizze der Serienschaltung von potenzialfreien Kontakten der Kontrollräume für einen Spillabbruch über einen Spillabbruchmagneten des Beschleunigungsbereichs;

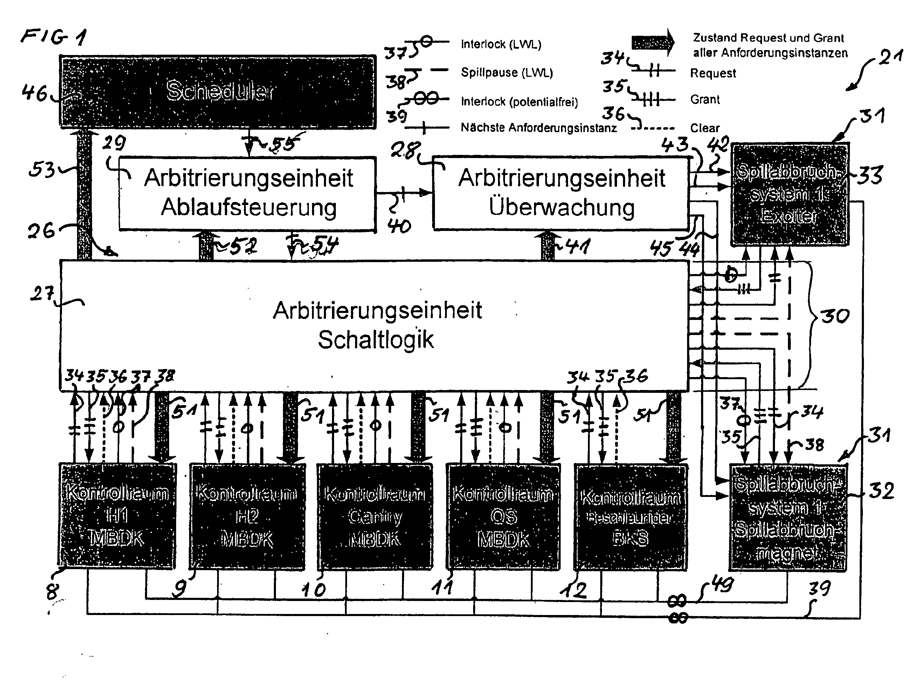

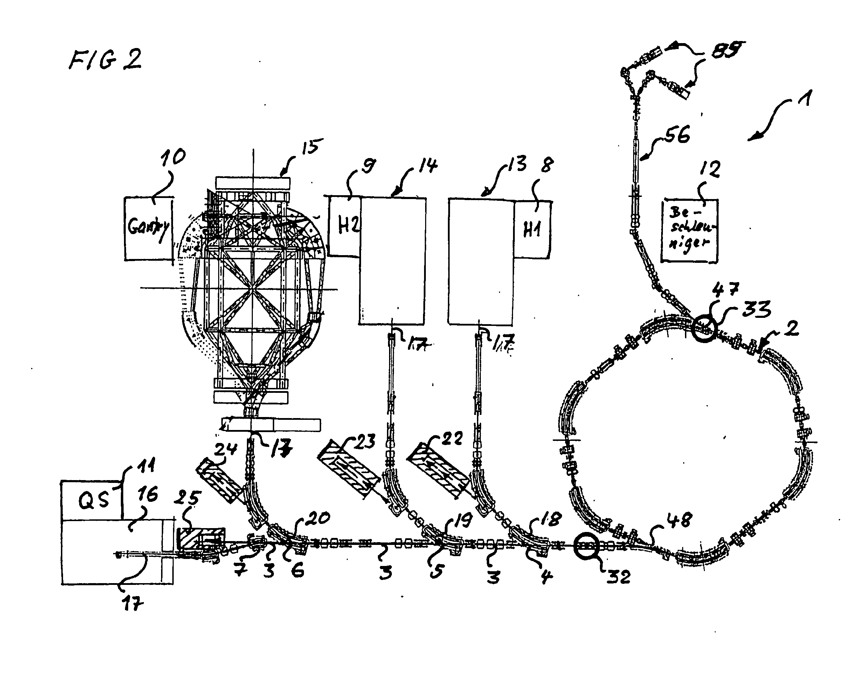

Figur 1 zeigt ein Schema eines Blockschaltbildes einer Strahlzuleitungsvorrichtung 21. Von der Strahlzuteilungsvorrichtung 21 bildet eine Arbitrierungseinheit 26 mit drei Schaltblöcken, nämlich einer Schaltlogik 27, einer Überwachungseinheit 28, und einer Ablaufsteuerung 29. Die Kontrollräume 8-12 von unterschiedlichen Anforderungsinstanzen sind über Busleitungen 51 und Spezialleitungen 34-38 mit der Schaltlogik 27 elektrisch verbunden. Die unterschiedlichen Anforderungsinstanzen sind drei Behandlungsräume, die in Figur 2 mit den Bezugszeichen 13-15 gekennzeichnet sind, wovon der Behandlungsraum 15 zu einem Gantry gehört, mit welchem der Einstrahlwinkel in den Patienten für den Ionenstrahl um 360° verändert werden kann. Ein weiterer Kontrollraum 11 ist für eine Qualitätssicherung QS in einem in Figur 2 gezeigten Qualitätssicherungsraum 16 vorgesehen, und ein fünfter Kontrollraum 12 ist dem Beschleuniger zugeordnet.

Die Schaltlogik 27 der Arbitrierungseinheit 26 hat über Signalleitungen 30 den Zugriff auf das Spillabbruchsystem 31 des Beschleunigungsbereichs. Das Spillabbruchsystem 31 des Beschleunigungsbereichs weist zwei Komponenten auf, nämlich einen Exciter 33 und dem Spillabbruchmagneten 32 zu denen jeweils die Signalleitungen 34, 35, 37 und 38 führen.

Die Überwachungseinheit 28 der Arbitrierungseinheit 26 verfügt über eine Eingangssignalverbindung 40 von der Ablaufsteuerung 29 der Arbitrierungseinheit 26 und über Eingangssignalverbindungen 41 in Form eines Signalbusses von der Schaltlogik 27 der Arbitrierungseinheit 26. Ferner verfügt die Überwachungseinheit 28 der Arbitrierungseinheit 26 über vier Ausgangssignalverbindungen 42 - 45. Die Ablaufsteuerung 29 der Arbitrierungseinheit 26 steht über die Eingangssignalverbindung 40 der Überwachungseinheit 28 mit dieser in Verbindung, und ist mit der Schaltlogik 27 der Arbitrierungseinheit 26 über einen Bus 52 und eine Ausgangssignalleitung 54 verbunden.

Während die bisher genannten Signalleitungen sicherheitsrelevant im Sinne des Medizingesetzes sind, steht die Arbitrierungseinheit 26 dieser Strahlzuteilungsvorrichtung 21 mit einem Scheduler 46 in Verbindung, der über eine Busleitung 53 mit der Schaltlogik 27 verbunden ist, und über eine Ausgangssignalleitung 55 in die Ablaufsteuerung 29 der Arbitrierungseinheit 26 eingreifen kann. Diese Signalleitungen 53 und 55 und auch der Scheduler 46 selbst sind nicht sicherheitsrelevant, sodass die Arbitrierungseinheit 26 bei Fehlen eines Schedulers 46, oder bei Versagen eines Schedulers 46 den Kontrollräumen B-12 reihum Gelegenheit gibt, Signale mit der Arbitrierungseinheit 26 bzw. der Schaltlogik 27 der Arbitrierungseinheit 26 über die Signalbusse 51 und die Spezialleitungen 34-38 auszutauschen.

Ferner hat jeder Kontrollraum über die redundanten Signalpfade 39 und 49 einen unmittelbaren Zugriff zu dem Spillabbruchsystem 31, wobei der redundante Signalpfad 49 Zugriff auf den Spillabbruchmagneten 32 und der redundante Signalfallpfad 39 einen Zugriff auf den Exciter 33 für die Kontrollräume 8-12 bereitstellt. Um zu gewährleisten, dass nicht gleichzeitig mehrere Kontrollräume 8 bis 12 auf das Spillabbruchsystem 31 zugreifen, sind zugriffsschaltelemente in den Kontrollräumen 8-12 auf die redundanten Signalpfade 39 und 49 in Reihe geschaltet, sodass bei Auslösung einer Unterbrechung der Zugriffsschaltelemente durch einen der Kontrollräume 8 bis 12 die übrigen Kontrollräume nicht mehr auf das Spillabbruchsystem 31 zugreifen können.

Das Spillabbruchsystem 31 verwendet bei dieser Bestrahlungstechnik insgesamt 4 Elemente, von denen im Blockschaltbild jedoch nur 2 Elemente, nämlich der Exciter 33 und der Spillabbruchmagnet 32 gezeigt werden. Zwei dieser Elemente (je zwei Umlenkmagnete pro Bestrahlungsplatz) gehören nicht zum Beschleunigungsbereich, sondern sind Ablenkkomponenten 4 bis 7, wie sie in Figur 2 gezeigt werden, die den Strahl von einer Transportstrecke in Richtung auf den jeweiligen anfordernden Bestrahlungsraum ablenken. Sie werden folglich direkt von dem jeweiligen Kontrollraum 8 bis 12 eines Bestrahlungsraumes bestätigt. Dazu werden sie direkt vom jeweiligen Kontrollraum 8 bis 10 eines Bestrahlungsraumes für einen Abbruch angesteuert, mit dem eine Bestrahlung durchzuführen ist.

Die anderen beiden Elemente, die im Blockschaltbild mit dem Exciter 33 und dem Spillabbruchmagneten 32 gezeigt werden, sind jedoch in dem Bereich der Strahlführung angeordnet, der allen Bestrahlungsstationen gemeinsam ist, und werden deshalb wechselweise der Kontrolle der unterschiedlichen Bestrahlungsräume unterworfen. Die Umschaltung der Kontrolle erfolgt durch die Arbitrierungseinheit 26, wobei die Zuteilung des Spillabbruchsystems 31, wie es das Blockschaltbild der Figur 1 zeigt, hochgradig sicherheitsrelevant im Sinne des Strahlenschutzgesetzes für medizinische Anlagen ist. Deshalb sind in dem Blockschaltbild der Figur 1 die Anordnung und Verteilung der Signalleitungen 34-38 explizit benannt, wobei das Bezugszeichen 34 eine Requestleitung, das Bezugszeichen 35 eine Grantleitung, das Bezugszeichen 36 eine Clearleitung, das Bezugszeichen 37 eine Interlockleitung und das Bezugszeichen 38 eine Spillpauseleitung kennzeichnen.

Der Scheduler 46 des Blockschaltbildes der Figur 1 gehört nicht zu den hochgradig sicherheitsrelevanten Komponenten der Strahlzuteilungsvorrichtung 21, jedoch besitzt er eine enge Verbindung aus logischer Sicht, zu der Arbitrierungseinheit 26. Die geplante Abfolge der Bestrahlungen wird durch eine Warteschlange bestimmt, die von einem zentralen Rechner der Bestrahlungstechnik, dem so genannten Schedulingrechner, verwaltet wird. Die zugehörige Schedulingapplikation ist von allen 4 lokalen Kontrollräumen 8 bis 11, dem Beschleunigerkontrollraum 12 und von diversen anderen Rechnern eines Kliniknetzes zugänglich.

Die geplante Abfolge der Bestrahlungen verändert sich ständig im Laufe eines Tages. Dazu überprüft die Schedulingapplikation bei jeder Änderung durch den Benutzer und nach jeder Bestrahlung die bestehende Abfolge, ändert sie gegebenenfalls und korrigiert entsprechend die Schätzungen für den geplanten Bestrahlungsbeginn eines Feldes. Diese Änderungen bewegen sich normalerweise nur im Bereich weniger Minuten eines Tages. Unter diesen Bedingungen macht der Scheduler 46 der Arbitrierungseinheit 26 über seine Ausgangssignalleitung 55 eine vorgabe an die Ablaufsteuerung 29 der Arbitrierungseinheit 26 für die jeweils nächste Zuteilung der Strahlhoheit.

Die Benutzeranforderung der Zuteilung des Strahls erfolgt vom jeweiligen Kontrollraum 8-12 aus. Diese Anforderung wird von der Arbitrierungseinheit 26 quittiert. Das Spillabbruchsystem 31 muss dann für diesen Bestrahlungsplatz des Kontrollraums, beispielsweise des Kontrollraumes 8, reserviert werden. Die Zuteilung des Strahls muss vom jeweiligen Kontrollraum 8-12 zurückgesetzt werden. Ohne quittierte Zuteilungsrechte wird die Bestrahlung nicht gestartet. Während der Bestrahlung können und dürfen die Zugriffsrechte nicht zurückgegeben werden. Nach Beendigung der Bestrahlung müssen die Zugriffsrechte jedoch wieder aktiv von dem Kontrollraum 8 an die Arbitrierungseinheit 26 zurückgegeben werden.

Wenn eine Schedulingapplikation nicht zur Verfügung steht, wird dennoch der Betrieb über die Arbitrierungseinheit 26 aufrecht erhalten. Steht eine Schedulingapplikation zur Verfügung, so werden die Zuteilungsrechte gemäß dieser Applikation vergeben. Falls dies jedoch nicht möglich ist, erfolgt die Vergabe nach dem verfahren, bei dem die Arbitriervngseinheit 26 den Kontrollräumen 8-12 reihum die Gelegenheit gibt, Zugriffsrechte zu reservieren. Außerdem wird dem Beschleunigerkontrollsystem bzw. dem Beschleunigerkontrollraum 12 das erteilte Zugriffsrecht eines Bestrahlungsplatzes mitgeteilt. Aus Sicherheitsgründen erhalten die Anforderungsinstanzen und ihre Kontrollräume 8-12 keine Zugriffsrechte, wenn die Arbitrierungseinheit 26 ausgeschaltet oder außer Funktion ist. Das bedeutet, die gesamte Anlage steht. Diese Maßnahme ist erforderlich, weil die Arbitrierungseinheit 26 das Spillabbruchsystem 31 des Beschleunigungsbereichs verwaltet. Ohne zugriff auf dieses schnelle Abbruchsystem ist weder eine Bestrahlung zulässig noch aufgrund der erfindungsgemäßen Arbitrierungseinheit 26 möglich. Mit der Arbitrierungseinheit 26 ist außerdem eine Testmöglichkeit zur Funktionsverifikation vorgesehen.

Da auch der Beschleuniger mit dem Kontrollraum 12 von der Arbitrierungseinheit 26 die Kontrolle über das Spillabbruchsystem 31 zugesprochen bekommen kann, werden im Folgenden die Kontrollräume 8 bis 12 auch Anforderungsinstanzen genannt. In diesem Ausführungsbeispiel der Erfindung steht der Begriff der Anforderungsinstanzen für die Gesamtheit aus vier Bestrahlungsplätzen, so weit sie unter Kontrolle der Kontroll- und sicherheitssysteme der Bestrahlungstechnik stehen, und dem Beschleuniger als fünfte Anforderungsinstanz. Dabei umfasst die Kontrolle des Beschleunigers alle explizit für den Beschleuniger freigegebenen Teile der Strahlführung und alle Bestrahlungsplätze, die unter Kontrolle des Beschleunigerkontrollsystems stehen.

Ein Ablauf einer Reservierung im Hinblick auf das Blockschaltbild der Figur 1 weist die nachfolgenden Schritte auf.
- Wenn eine Anforderungsinstanz bzw, ein Kontrollraum 8 bis 12 grundsätzlich alle Vorkehrungen getroffen hat, eine Bestrahlung zu beginnen, dann wird entweder auf Druck auf einen Knopf einer Konsole des Kontrollraums, oder durch ein von einem Rechner generiertes Signal eine Anforderung an die Arbitrierungseinheit 26 in Form eines Requestsignals über die Requestleitung 34 übermittelt. Insbesondere die Benutzer in dem Kontrollraum des bestrahlungsaktiven Behandlungsraumes, aber auch das Personal in anderen Kontrollräumen 8-12, erhalten eine optische Anzeige dieser Anforderung.
- Damit die Anforderungsinstanz die Hoheit über das Spillabbruchsystem 31 erhält, sind mehrere Bedingungen zu erfüllen :
   - Es hat zunächst keine andere Anforderungsinstanz die Hoheit über das Spillabbruchsystem 31 des Beschleunigungsbereichs. Die Hoheit über das Spillabbruchsystem 31 wird erst neu vergeben, wenn die aktuelle Anforderungsinstanz mit ihrem bestrahlungsaktiven Bestrahlungsraum selber aktiv die Kontrolle abgibt.
   - Der Scheduler 46 hat die Anforderungsinstanz als nächstes Ziel vorzugeben. Falls der Scheduler jedoch deaktiviert ist, werden die Zugriffsrechte reihum den Anforderungsinstanzen von der Arbitrierungseinheit 26 angeboten.
   - Das Spillabbruchsystem 31 muss bereitstehen, damit eine Anforderungsinstanz die Hoheit über das Spillabbruchsystem 31 erhält.
- Die erfolgreiche Reservierung für einen Zugriff auf das Spillabbruchsystem 31 wird von beiden Elementen, nämlich dem Exciter 33 und dem Spillabbruchmagneten 32 des Spillabbruchsystems 31 quittiert, in Form eines Grantsignals über die Grantleitungen 35. Insbesondere die Benutzer des lokalen Kontrollraums, aber auch das Personal in allen anderen Kontrollräumen B-12, erhalten wiederum eine optische Anzeige dieser Reservierung.
- Während der laufenden Bestrahlung, das heißt, wenn ein Bestrahlungsraum bestrahlungsaktiv ist, kann die Anforderungsinstanz die beiden Komponenten des Spillabbruchsystems 31 mithilfe einer Spillpauseleitung 38 und alternativ über einen redundanten Signalpfad 39 bzw. 49 einer Interlockleitung jederzeit abschalten.
- Nach Ende einer Bestrahlung wird die Anforderung bzw. der "Request" von der Anforderungsinstanz bzw. den Kontrollräumen 8-12 explizit wieder zurückgenommen mit einem Clearsignal über die Clearleitung 36. Dieses löscht gleichzeitig auch das Grantsignal.

Figur 2 zeigt eine Prinzipskizze eines Teilchenbeschleunigers 1 mit Komponenten, auf die von der Strahlzuteilungsvorrichtung 21 gemäß Figur 1 zurückgegriffen wird. Der Teilchenbeschleuniger 1 weist einen Beschleunigungsbereich 2 in Form eines Synchrotrons auf, der Ionenpakete auf Energien von etwa 4500 MeV beschleunigt. Diese Ionenpakete werden in einer Ionenquelle 89 erzeugt und über einen Linearbeschleuniger 56 vorbeschleunigt, sowie in einem Einkopplungsbereich 47, in dem auch der Exciter 33 angeordnet ist, in den Beschleunigerring 2 eingekoppelt.

Der Exciter 33 ist eine Hochfrequenzresonanzkomponente in diesem Einkopplungsbereich 47 des Beschleunigers 2 und kann durch verstellen bzw. Fehlanpassen der Resonanzabstimmung einen Strahlabbruch in Mikrosekunden auslösen. In einem Auskopplungsbereich 48 des Beschleunigerringes 2 ist der Spillabbruchmagnet 32, der genauso wie der Exciter 33 für einen schnellen Strahlabbruch bei einer Ionenstrahlbehandlung im Rasterscanverfahren sicherheitsrelevant einsetzbar ist, angeordnet. In einer Transportstrecke 3 des Teilchenstrahls 17 sind für jeden Bestrahlungsraum 13-15 Ablenkmagnete 18 bis 20 angeordnet, die den Ionenstrahl 17 zu den Bestrahlungsräumen 13-15 ablenken. Den Bestrahlungsräumen 13-15 sind die Kontrollräume 8-10 zugeordnet, die direkt auf die Ablenkmagnete 18-20 einwirken können, und einen Strahlabbruch zu den Auffangkammern 22-25 in einigen hundert Millisekunden bewirken können. Diese Ablenkkomponenten 4-7 der Transportstrecke 3 sind damit nicht so schnell, wie die Strahlabbruchmöglichkeiten durch die Spillabbruchkomponenten 32 und 33 im Bestrahlungsring 2.

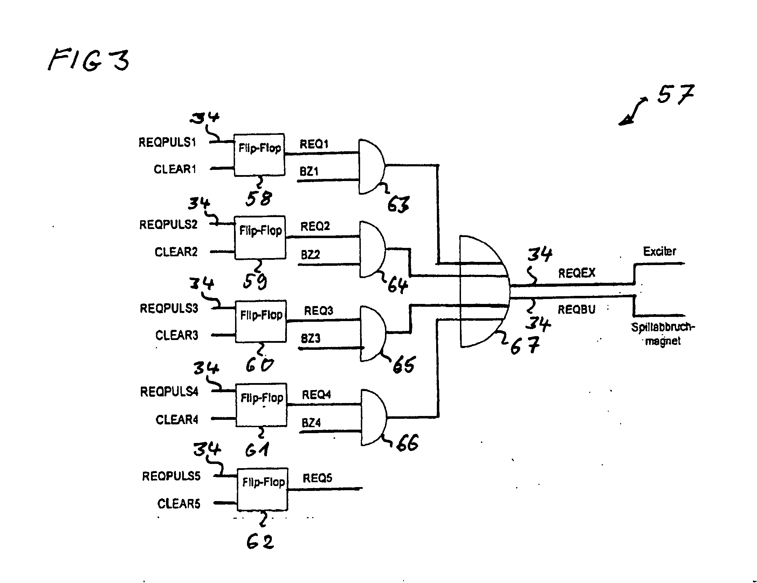

Figur 3 zeigt eine Prinzipskizze einer Requestschaltung 57 zur Verarbeitung von Requestsignalen REQPULS1-REQPULS5 über Requestleitungen 34 von den in Figur 1 und 2 gezeigten Kontrollräumen 8-12. Die Requestschaltung 57 sorgt dafür, dass jeweils nur das Requestsignal der gerade aus Sicht der Ablaufsteuerung der Arbitrierungseinheit aktiven Anforderungsinstanz bzw. des aktiven Kontrollraums an den in Figur 1 und 2 gezeigten Exciter 33 bzw. Spillabbruchmagneten 32 weitergeleitet wird.

Wenn die Anforderungsinstanz der Kontrollraum des Beschleunigers selbst ist, wird keine Reservierung vorgenommen, da Exciter und Spillabbruchmagnet ohnehin durch den Beschleuniger steuerbar sind. Demgemäß werden an Flip-Flops 58 bis 62 der Schaltlogik der Arbitrierungseinheit die Requestsignale REQPULS1 bis REQPULS5 angelegt, wobei die Flip-Flops 58-62 vorher über die Eingänge CLEAR1 - CLEAR5 auf neutral zurückgesetzt waren. Die Ausgänge der Flip-Flops 58-61 werden logischen ANDGATEs 63-66 zugeführt und dabei über zweite Eingänge BZ1 bis BZ4 der ANDGATEs 63-66 nur die Requestanforderungen weitergegeben, die mit dem zweiten Eingang ein bestätigtes, aktives Strahlziel BZ aufweisen. Die Ausgänge der ANDGATEs 63-66 werden einem ORGATE 67 zugeführt, dessen Ausgänge REQEX und REQBU jeweils über eine Requestleitung 34 mit dem Exciter bzw. dem Spillabbruchmagneten des Spillabbruchsystems des Beschleunigerringes verbunden sind,

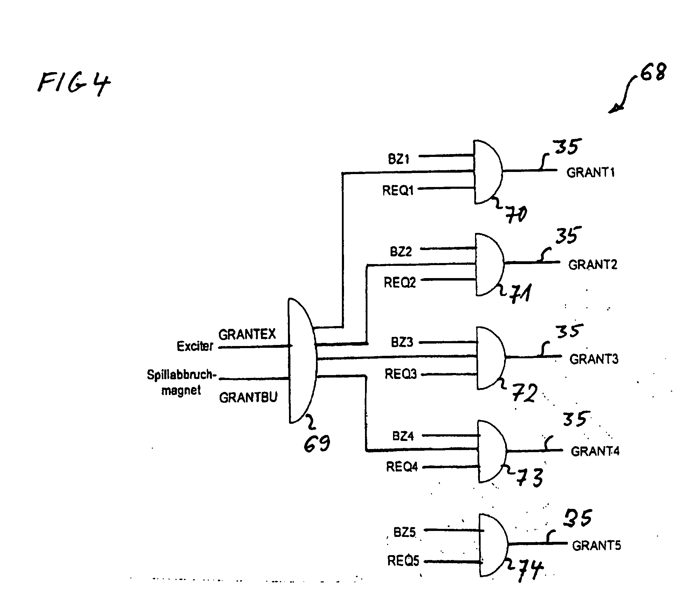

Figur 4 zeigt eine Prinzipskizze einer Grantschaltung 68, die nun von Grantleitungen GRANTEX und GRANTBU von dem Exciter bzw. dem Spillabbruchmagneten des Spillabbruchsystems über ein ANDGATE 69 an entsprechende ANDGATEs 70-73 weitergegeben werden, die neben diesem Eingang von dem ANDGATE 69 zwei weitere Eingänge aufweisen, nämlich BZ1 bis BZ4 und REQ1 bis REQ4, die sicherstellen, dass sowohl ein Requestsignal an einem der Eingänge REQ1 bis REQ4, als auch ein Signal für ein bestätigtes, aktives Strahlziel an einem der Eingänge BZ1 bis BZ4 der vier ANDGATEs 70-73 anliegt, sodass am Ausgang dieser ANDGATEs ein Signal GRANT1 bis GRANT4 über entsprechende Grantleitungen 35 zu den Kontrollräumen zurückgegeben werden kann. Das Grantsignal GRANT1 bis GRANT5 setzt ein Request an den Eingängen REQ1 bis REQ5 der ANDGATEs 70-74 und eine Zuteilung durch die Arbitrierungseinheit an den Eingängen BZ1 bis BZ5 der ANDGATEs 70-74 voraus. Die Überprüfung des Requestsignals ist an dieser Stelle redundant, da die Zuteilung durch die Arbitrierungseinheit ein Request der Anforderungsinstanz bzw. der Kontrollräume voraussetzt. Der Beschleuniger benötigt kein Grantsignal vom Spillabbruchelement, sondern lediglich die Zuteilung durch die Arbitrierungseinheit, die jedoch ebenfalls über die Grantleitung GRANT5 signalisiert wird. Es ist somit durch diese Grantschaltung sicherheitsrelevant gewährleistet, dass jeweils nur eine Anforderungsinstanz bzw. ein Kontrollraum von der Arbitrierungseinheit mit einem Grantsignal über die Grantleitungen 35 zu einem der Kontrollräume gegeben wird.

Figur 5 zeigt eine Prinzipskizze einer Spillpause-Schaltung 75. Diese Spillpause-Schaltung 75 mit den Spillpauseleitungen 38 kann von allen vier Kontrollräumen der drei Bestrahlungsräume und dem einen Qualitätssicherungsraum nur dann verwendet werden, wenn ein Grantsignal GRANT1 bis GRANT4 der Spillabbruchelemente des Spillabbruchsystems vorliegt. Demgemäß weist die Spillpause-Schaltung 75 vier ANDGATEs 76-79 auf, die jeweils zwei Eingänge für ein Grantsignal GRANT1 bis GRANT4 und ein Spillsignal SPILL1 bis SPILL4 aufweisen, wobei diese Signale über Grantleitungen 35 und Spillpauseleitungen 38 an den ANDGATEs 76-79 anliegen. Die Ausgänge der ANDGATEs 76-79 führen zu Eingängen eines ORGATEs 80, das zwei Ausgänge SPILLEX und SPILLBU aufweist, die mit dem Exciter bzw. dem Spillabbruchmagneten über jeweils eine Spillpausleitung 38 verbunden sind. Wenn der Beschleuniger die Hoheit hat, steuert er den Exciter und den Spillabbruchmagneten direkt an. Die Spillpause-Leitung 38 wird also vom Beschleuniger nicht verwendet. Mit der Spillpause-Schaltung 75 wird nicht unterschieden, ob eine Spillpause oder bereits ein Spillende vorliegt. Die Entscheidung darüber, ob der Reststrahl im Beschleunigungsbereich, dem Synchrotron, vernichtet werden soll, trifft allein das Beschleunigerkontrollsystem in dem entsprechendem Kontrollraum.

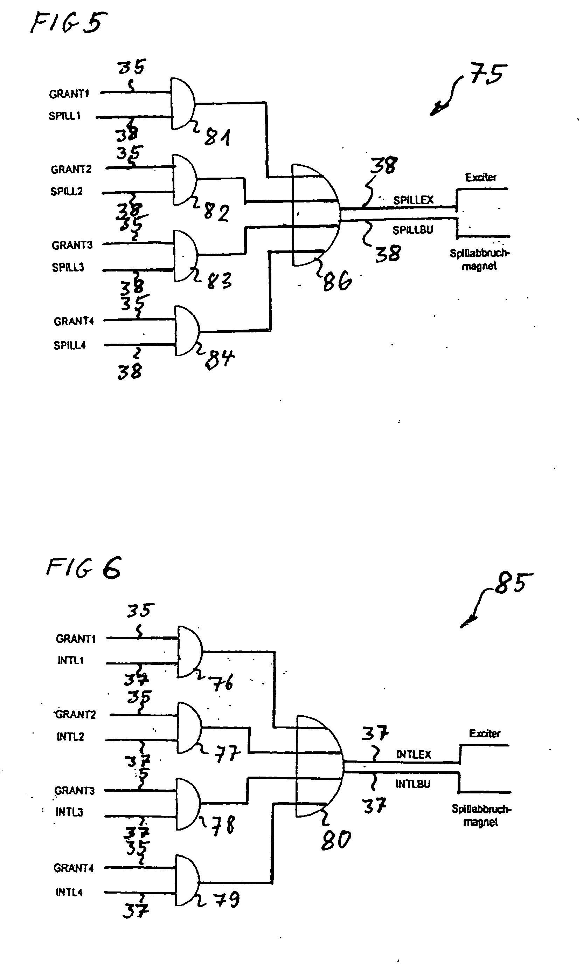

Figur 6 zeigt eine Prinzipskizze einer Interlock-Schaltung 85 zur Verarbeitung von Interlocksignalen INTL1 bis INTL4 über Interlockleitungen 37 durch ANDGATEs 81-84 an deren Eingängen gleichzeitig ein Grantsignal GRANT1 bis GRANT4 über die Grantleitungen 35 anliegen muss, um über einen der Ausgänge der ANDGATEs 81-84 an den Eingang eines ORGATEs 86. Das ORGATE 86 weist wiederum zwei Ausgänge INTLEX und INTLBU auf, die zum Exciter bzw. zum Spillabbruchmagneten über Interlockleitungen 37 führen. Somit kann die Interlockleitung 37 des Exciters und des Spillabbruchmagneten von allen vier Bestrahlungsplätzen nur dann verwendet werden, wenn das Grantsignal GRANT1 bis GRANT4 der Spillabbruchelemente vorliegt. Hat der Beschleuniger die Hoheit, steuert er den Exciter und den Spillabbruchmagneten direkt an, sodass die Interlockleitung 37 vom Beschleuniger nicht verwendet wird.

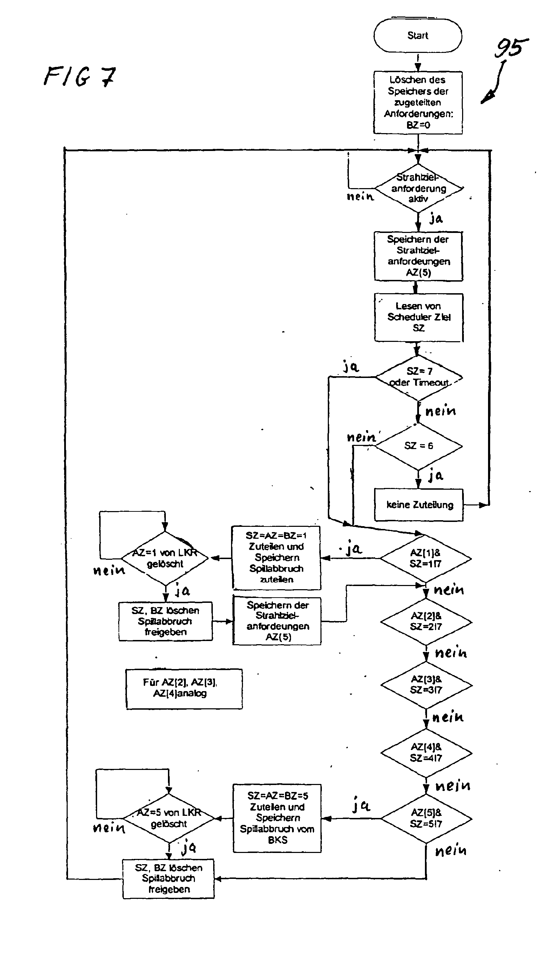

Figur 7 zeigt ein Schema eines Flussdiagramms 95 einer Ablaufsteuerung einer Arbitrierungseinheit. In diesem Flussdiagramm 95 wird berücksichtigt, dass ein Scheduler vorhanden sein kann, oder auch nicht. Entsprechend ändert sich der Durchlauf durch das Flussdiagramm 95. In dem Flussdiagramm 95 wird die Strahlzielvorgabe des Schedulers mit dem Bezugszeichen SZ definiert, das anfordernde Strahlziel bzw. der anfordernde Kontrollraum mit AZ gekennzeichnet und ein bestätigtes, aktives Strahlziel wird mit dem Bezugszeichen BZ angegeben. Diese Ablaufsteuerung erhält die Arbitrierungseinheit vom Scheduler über ein digitales Ein-/Aus-Modul, die gewünschte nächste Anforderungsinstanz bzw. den nächsten anfordernden Kontrollraum.

Die Anforderungsinstanzen, die im Flussdiagramm 95 mit AZ1 bis AZ4 gekennzeichnet werden, entsprechen den Bestrahlungsräumen H1, H2, Gantry und QS in dem in Figur 1 gezeigten Blockschaltbild. Die Anforderungsinstanz AZ5 entspricht dem Beschleuniger zu dem die Strahlführung bis zum Strahlvernichter des Beschleunigers und alle für den Beschleuniger freigegebenen Bestrahlungsplätze gehören. Die Anforderungsinstanz SZ6 bedeutet, dass in dem Moment keine Vergabe der Hoheit erfolgt, und die Anforderungsinstanz SZ7 bedeutet, dass der Scheduler nicht aktiv ist, und keine Vorgaben liefert, sodass die Arbitrierungseinheit selbstständig die Hoheit vergibt. Diese Vergabe durch die Arbitrierungseinheit soll dann nach einem Rundum-Verfahren erfolgen, bei dem die Arbitrierungseinheit den Anforderungseinheiten bzw. Kontrollräumen reihum die Gelegenheit, eine Reservierung vorzunehmen, ermöglicht.

Die Verbindung zwischen Scheduler und Arbitrierungseinheit ist durch geeignete reservierte Direktleitungen oder über andere Mechanismen insoweit abgesichert, dass die Arbitrierungseinheit erkennt, wenn der Scheduler nicht aktiv ist, und somit die gelesenen Anforderungsinstanzen in jedem Fall ungültig sind. In einem solchen Fall wird so verfahren, als sei die Anforderungsinstanz 7 gelesen worden, die einer Aufforderung entspricht, nach einem Reihum-Verfahren vorzugehen. Zusätzlich wird das Fehlen der Schedulersignale optisch an der Arbitrierungseinheit angezeigt und ebenso wird der Zustand aller Request-, Grant- und Interlockleitungen und die gewählte Anforderungsinstanz bei einem laufenden Scheduler visuell sichtbar gemacht. Ferner verfügen die Arbitrierungseinheiten über einen lokalen Modus, in dem grundsätzlich kein Versuch unternommen wird, eine Anforderungsinstanz bzw. ein Kontrollraum vom Scheduler zu lesen, sodass ein lokaler Schalter ebenfalls eine Vergabe in dem Reihum-Verfahren bewirkt. Der Zustand des lokalen Schalters im jeweiligen Kontrollraum wird optisch ebenfalls am Gerät angezeigt.

Das Flussdiagramm 95 für die Ablaufsteuerung ergänzt die Verarbeitung von Request-, Grant- und Interlocksignalen, die oben beschrieben wurden. Die Ablaufsteuerung der Arbitrierungseinheit liest entsprechend dem Flussdiagramm 95 die vom Scheduler gewünschte Anforderungsinstanz bzw. den entsprechenden Kontrollraum, die auch als Scheduler-Instanz SI bezeichnet wird, ein. Sobald die Strahlhoheit neu vergeben wird, wird die Bestrahlungsinstanz BI an die Request- und Grant-Schaltungen weitergegeben und sorgt auf diesem Wege dafür, dass nur der gewählte Platz die Spillabbruchelemente reservieren kann. Sobald die Reservierung erfolgt ist, nimmt die Ablaufsteuerung der Arbitrierungseinheit keinen Einfluss mehr vor. Insbesondere wird das Interlocksignal nicht durch die Ablaufsteuerung beeinflusst.

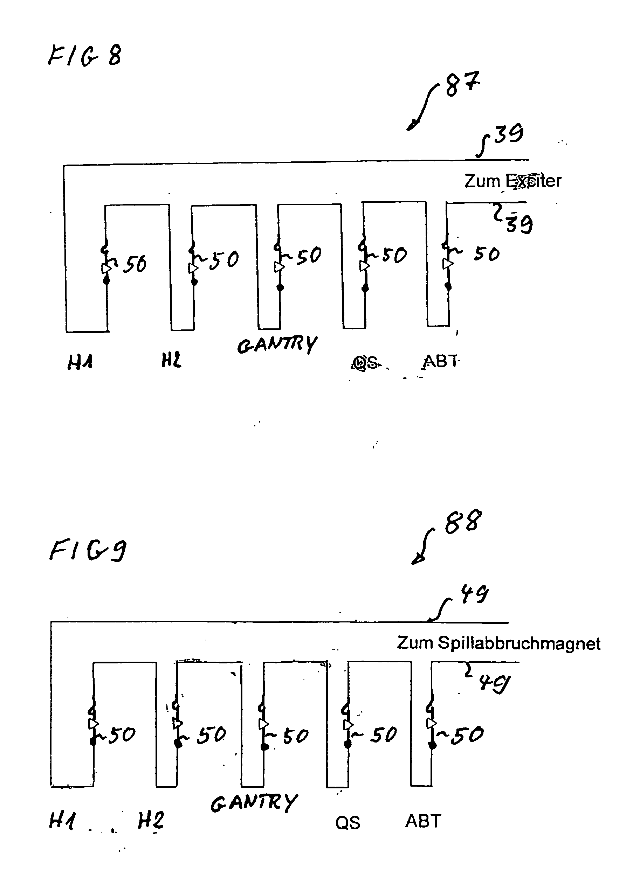

Figur 8 zeigt eine Prinzipskizze der Serienschaltung 87 von potenzialfreien Kontakten 50 der Kontrollräume für einen Spillabbruch über einen Exciter des Beschleunigungsbereichs. Über einen redundanten Signalpfad 39 wird der Exciter direkt von einem der Kontrollräume angesteuert, um innerhalb von wenigen Mikrosekunden einen Strahlabbruch zu bewirken. Dazu liegen in dem redundanten Signalpfad 39 Unterbrecherkontakte 50 für die drei Kontrollräume mit Bestrahlungsräumen H1, H2 und GANTRY, sowie für den Qualitätssicherungsraum QS und für die Arbitrierungseinheit ABT, die ebenfalls über den redundanten Signalpfad 39 den Exciter für einen schnellen Strahlabbruch vorsehen kann.

Figur 9 zeigt eine Prinzipskizze der Serienschaltung 88 von potenzialfreien Kontakten 50 der Kontrollräume für einen Spillabbruch über einen Spillabbruchmagneten des Beschleunigungsbereichs. Die potenzialfreien Kontakte 50 des Spillabbruchsystems sind somit pro Bestrahlungsraum bzw. Kontrollraum zweifach für Exciter und Spillabbruchmagnet vorhanden. Sie können nur dann den Spillabbruch hervorrufen, wenn an dem entsprechenden Bestrahlungsplatz eine Bestrahlung gestartet wurde, jedoch nicht, wenn keine Bestrahlungsaktivitäten im Bestrahlungsraum stattfinden. Dazu sind die einzelnen potenzialfreien Kontakte 50 der Bestrahlungsplätze in Reihe geschaltet und sind von dem Arbitrierungsmechanismus selbst unabhängig. Doch hat die Arbitrierungseinheit die Möglichkeit, auch auf diese Weise über einen Spillabbruch über die redundanten Signalpfade 39 oder 49 auszulösen.

### Bezugszeichenliste

- 1: Teilchenbeschleuniger
- 2: Beschleunigerring
- 3: Transportstrecke
- 4-7: Ablenkkomponente
- 8: Kontrollraum H1
- 9: Kontrollraum H2
- 10: Kontrollraum Gantry
- 11: Kontrollraum Q2
- 12: Kontrollraum, Beschleuniger
- 13: Behandlungsraum
- 14: Behandlungsraum
- 15: Behandlungsraum (Gantry)
- 16: Qualitätssicherungsraum
- 17: Teilchenstrahl
- 18-20: Ablenkmagnet
- 21: Strahlzuteilungsvorrichtung
- 22-25: Auffangkammer
- 26: Arbitrierungseinheit
- 27: Schaltlogik
- 28: Überwachungseinheit
- 29: Ablaufsteuerung
- 30: Signalleitung
- 31: Spillabbruchsystem
- 32: Spillabbruchmagnet
- 33: Exciter
- 34: Requestsignalleitung
- 35: Grantsignalleitung
- 36: Clearsignalleitung
- 37: Interlockleitung
- 38: Spillpauseleitung
- 39: redundanter Signalpfad zum Exciter
- 40: Eingangssignalverbindung der Überwachungseinheit
- 41: Eingangssignalverbindung der Überwachungseinheit
- 42: Interlocksignal der Überwachungseinheit
- 43: Interlocksignal (potentialfrei) der Überwachungseinheit
- 44: Interlocksignal der Überwachungseinheit
- 45: Interlocksignal (potentialfrei) der Überwachungseinheit
- 46: Scheduler
- 47: Einkopplungsbereich
- 48: Auskopplungsbereich
- 49: redundanter Signalpfad zum Spillabbruchmagneten
- 50: Schaltelemente eines Kontrollraums
- 51: Signalbus der Kontrollräume
- 52: Signalbus der Ablaufsteuerung
- 53: Signalbus des Schedulers
- 54: Ausgangssignalleitung der Ablaufsteuerung
- 55: Ausgangssignalleitung des Schedulers
- 56: Linearbeschleuniger
- 57: Requestschaltung
- 58-62: Flip-Flop der Request-Schaltung
- 63-66: ANDGATEs der Request-Schaltung
- 67: ORGATE der Request-Schaltung
- 68: Grant-Schaltung
- 69: ANDGATE der Grant-Schaltung
- 70-74: ANDGATEs der Grant-Schaltung
- 75: Spillpause-Schaltung
- 76-79: ANDGATEs der Spillpause-Schaltung
- 80: ORGATE der Spillpause-Schaltung
- 81-84: ANDGATEs der Interlock-Schaltung
- 85: Interlock-Schaltung
- 86: ORGATE der Interlock-Schaltung
- 87: Serienschalter zum Exciter
- 88: Serienschalter zum Spillabbruchmagnet
- 89: Ionenquelle

- 95: Flussdiagramm

## Patentansprüche

1. Strahlzuteilungsverfahren für medizinische Teilchenbeschleuniger (1), wobei der Teilchenbeschleuniger (1) mindestens einen Beschleunigungsbereich (2), eine Transportstrecke (3) und Ablenkkomponenten (4 bis 7) aufweist, über welche mehrere jeweils einen Kontrollraum (8 bis 10) aufweisende Behandlungsräume (13 bis 15) mit einem Teilchenstrahl (17) aus Ionenpaketen sequenziell versorgt werden, wobei das Strahlzuteilungsverfahren folgende Verfahrenschritte aufweist:
- Anfordern einer Reservierung der Teilchenstrahlhoheit insbesondere einer Reservierung eines direkten Zugriffs auf ein Spillabbruchsystem (31) des von allen Behandlungsräumen (13 bis 15) benötigten Beschleunigungsbereichs (2) für eine geplante Dauer einer Bestrahlung durch einen der Kontrollräume (8 bis 12);
- Durchführen einer sicherheitsrelevanten Reservierung des Spillabbruchsystems (31) durch eine elektronische Schaltlogik (27) einer Arbitrierungseinheit (26);
- Mitteilen der erfolgten Reservierung an jeden Kontrollraum (8 bis 12) unter Zulassen nur eines Kontrollraumes (8 bis 12) für einen Strahlabbruch über das Spillabbruchsystem (31);
- Bereithalten eines von der Arbitrierungseinheit (26) unabhängigen und unmittelbaren redundanten Signalpfades (39, 49) zum Spillabbruchsystem (31) des Beschleunigungsbereichs (2) für die Kontrollräume (8 bis 12), wobei die Schaltelemente (50) der Kontrollräume (8 bis 12) in Reihe geschaltet sind,
wobei die Arbitrierungseinheit (26) bei gleichzeitiger Strahlreservierungsanforderung mehrerer Kontrollräume (8 bis 12) entscheidet, welcher Kontrollraum (8 bis 12) eines Bestrahlungsplatzes (13 bis 15) die Reservierung vornehmen kann und
wobei trotz Verlust einer Reservierung ein Spillabbruch von jedem Kontrollraum (8 bis 12) über den redundanten Signalpfad (39, 49) ausgelöst werden kann.

2. Strahlzuteilungsverfahren nach Anspruch 1,
**dadurch gekennzeichnet, dass**
die Strahlhoheit erst dann von einem aktiven Kontrollraum (8 bis 12) zu einem anderen Kontrollraum (8 bis 12) übergeben wird, wenn der aktuelle Kontrollraum (8 bis 12) die Kontrolle über den Teilchenstrahl (17) aktiv abgibt.

3. Strahlzuteilungsverfahren nach Anspruch 1 oder Anspruch 2,
**dadurch gekennzeichnet, dass**
das Spillabbruchsystem (31) des Beschleunigerringes (2) für eine kurzzeitige Strahlabschaltung aktiviert wird.

4. Strahlzuteilungsverfahren nach einem der Ansprüche 1 bis 3,
**dadurch gekennzeichnet, dass**
ein Kontrollraum (12) für den Teilchenbeschleuniger (1) eine Reservierung für das Spillabbruchsystem (31) vornimmt, wenn gewährleistet ist, dass entweder kein Strahl zu den Bestrahlungsräumen (13 bis 15) gelangt oder dass sich dort kein Patient aufhält.

5. Strahlzuteilungsverfahren nach einem der Ansprüche 1 bis 4,
**dadurch gekennzeichnet, dass**
der Status aller Reservierungen, Zugriffsberechtigungen und Quittungen durch eine Überwachungseinheit (28) der Arbitrierungseinheit (26) überprüft wird und bei Inkonsistenz eine Abschaltung über den redundanten Signalpfad (39, 49) durchgeführt wird.

6. Strahlzuteilungsverfahren nach einem der Ansprüche 1 bis 5,
**dadurch gekennzeichnet, dass**
durch ein nicht sicherheitsrelevantes Mikroprozessorsystem als Scheduler (46) eine Abfolge der Vergabe von Reservierungen des Spillabbruchsystems (31) der Arbitrierungseinheit (26) mitgeteilt wird und bei Fehlen des Mikroprozessorsystems oder bei Fehlen von Vergaben die Arbitrierungseinheit (26) der Kontrollräume (8 bis 12) reihum die Gelegenheit gibt, eine Reservierung vorzunehmen.

## Claims

1. Beam allocation method for medical particle accelerators (1), the particle accelerator (1) having at least one acceleration region (2), a transport path (3) and deflection components (4 to 7), by way of which a plurality of treatment rooms (13 to 15), each having a control room (8 to 10), are sequentially supplied with a particle beam (17) comprising ion packets, the beam allocation method comprising the following method steps:
- requesting reservation, by one of the control rooms (8 to 12), of particle beam sovereignty, especially reservation of direct access to a spill abort system (31) of the acceleration region (2) required by all the treatment rooms (13 to 15), for a planned irradiation duration;
- carrying out safety-relevant reservation of the spill abort system (31) by an electronic switching logic (27) of an arbitration unit (26);
- reporting of the completed reservation to each control room (8 to 12) with permission for only one control room (8 to 12) to carry out beam aborting by means of the spill abort system (31);
- holding in a state of readiness, for the control rooms (8 to 12), a direct redundant signal path (39, 49), which is independent of the arbitration unit (26), leading to the spill abort system (31) of the acceleration region (2), the switching elements (50) of the control rooms (8 to 12) being connected in series, it being possible, in the event of simultaneous beam reservation requests from a plurality of control rooms (8 to 12), for the arbitration unit (26) to decide which control room (8 to 12) of an irradiation position (13 to 15) can make the reservation and,
it being possible, despite the loss of a reservation, for a spill abort to be initiated from each control room (8 to 12) by way of the redundant signal path (39, 49).

2. Beam allocation method according to claim 1,
**characterised in that**
beam sovereignty is transferred from an active control room (8 to 12) to another control room (8 to 12) only when the current control room (8 to 12) actively relinquishes control over the particle beam (17).

3. Beam allocation method according to claim 1 or claim 2,
**characterised in that**
the spill abort system (31) of the accelerator ring (2) is activated for switching off the beam for a short period.

4. Beam allocation method according to one of claims 1 to 3,
**characterised in that**
a control room (12) for the particle accelerator (1) makes a reservation for the spill abort system (31) once it has been ensured that either no beam is reaching the irradiation rooms (13 to 15) or that no patient is present therein.

5. Beam allocation method according to one of claims 1 to 4,
**characterised in that**
the status of all reservations, access entitlements and acknowledgements are checked by a monitoring unit (28) of the arbitration unit (26) and, in the event of inconsistency, switching-off is carried out by way of the redundant signal path (39, 49).

6. Beam allocation method according to one of claims 1 to 5,
**characterised in that**,
by means of a non-safety-relevant microprocessor system in the form of a scheduler (46), a sequence of reservation allocation of the spill abort system (31) is reported to the arbitration unit (26) and, when the microprocessor system is absent or when allocations are absent, the arbitration unit (26) gives the control rooms (8 to 12), in turn, the opportunity to make a reservation.

## Revendications

1. Méthode d'allocation de faisceaux pour accélérateurs médicaux de particules (1), l'accélérateur de particules (1) comprenant au moins une zone d'accélération (2), un trajet de transport (3) et des composants de déviation (4 à 7) par l'intermédiaire desquels plusieurs compartiments de traitement (13 à 15) qui comprennent respectivement un compartiment de contrôle (8 à 10) sont approvisionnés en mode séquentiel d'un faisceau de particules (17) se composant de paquets d'ions, sachant que la méthode d'allocation de faisceaux comprend les étapes de procédé suivantes :
- Demande d'une réservation de la prépondérance sur les faisceaux de particules par l'un des compartiments de contrôle (8 à 12), notamment d'une réservation d'un accès direct à un système d'interruption de déversement (31) de la zone d'accélération (2) dont ont besoin tous les compartiments de traitement (13 à 15) pour une durée planifiée pour une irradiation ;
- Attribution d'une réservation de sécurité du système d'interruption de déversement (31) par une logique électronique de commutation (27) d'une unité d'arbitrage (26) ;
- Notification de la réservation effectuée à chaque compartiment de contrôle (8 à 12) avec habilitation d'un seul compartiment de contrôle (8 à 12) pour une interruption de faisceaux par l'intermédiaire du système d'interruption de déversement (31) ;
- Tenue à disposition d'une voie de circulation de signaux (39, 49) qui est indépendante de l'unité d'arbitrage (26), qui est directement redondante et qui conduit vers le système d'interruption de déversement (31) de la zone d'accélération (2) pour les compartiments de contrôle (8 à 12), les éléments de commutation (50) des compartiments de contrôle (8 à 12) étant disposés en série,
alors qu'en cas demande simultanée de réservation de faisceaux émanant de plusieurs compartiments de contrôle (8 à 12), l'unité d'arbitrage (26) décide quel compartiment de contrôle (8 à 12) d'un lieu d'irradiation (13 à 15) peut effectuer la réservation et alors que, malgré la perte d'une réservation, une interruption du déversement peut être déclenchée par chaque compartiment de contrôle (8 à 12) par l'intermédiaire de la voie redondante de circulation de signaux (39, 49).

2. Méthode d'allocation de faisceaux selon la revendication 1, **caractérisée en ce que** la prépondérance sur les faisceaux n'est transmise d'un compartiment de contrôle actif (8 à 12) à un autre compartiment de contrôle (8 à 12) que si le compartiment de contrôle actuel (8 à 12) cède de manière active le contrôle sur le faisceau de particules (17).

3. Méthode d'allocation de faisceaux selon la revendication 1 ou la revendication 2, **caractérisée en ce qu'**une activation du système d'interruption de déversement (31) de l'anneau d'accélérateur (2) a lieu pour une interruption brève du faisceau.

4. Méthode d'allocation de faisceaux selon l'une quelconque des revendications 1 à 3, **caractérisée en ce qu'**un compartiment de contrôle (12) pour l'accélérateur de particules (1) effectue une réservation pour le système d'interruption de déversement (31) s'il est garanti soit qu'aucun faisceau n'arrive aux compartiments d'irradiation (13 à 15) soit qu'aucun patient ne séjourne à cet endroit.

5. Méthode d'allocation de faisceaux selon l'une quelconque des revendications 1 à 4, **caractérisée en ce que** le statut de toutes les réservations, autorisations d'accès et acquittements est vérifié par une unité de surveillance (28) de l'unité d'arbitrage (26) et **en ce qu'**en cas d'inconsistance une interruption a lieu par l'intermédiaire de la voie redondante de circulation de signaux (39, 49).

6. Méthode d'allocation de faisceaux selon l'une quelconque des revendications 1 à 5, **caractérisée en ce qu'**un système de microprocesseurs ne se rapportant pas à la sécurité et servant de programmateur (46) communique à l'unité d'arbitrage (26) une chronologie des concessions de réservations du système d'interruption de déversement (31) et **en ce qu'**en cas d'absence du système de microprocesseurs ou d'absence de concessions, l'unité d'arbitrage (26) donne aux compartiments de contrôle (8 à 12) tour à tour la possibilité d'effectuer une réservation.
